Europäisches Patentamt

European Patent Office

Office européen des brevets

(11) Publication number: **0 352 387 B1**

## EUROPEAN PATENT SPECIFICATION

(45) Date of publication of patent specification: **30.12.92**

(21) Application number: **88307984.0**

(22) Date of filing: **30.08.88**

(51) Int. Cl.5: **C12N 15/62**, C12P 21/02,
C12N 1/20, //(C12N1/20,
C12R1:125)

(54) Method for preparing a hirudin.

(30) Priority: **26.07.88 JP 184557/88**

(43) Date of publication of application:
**31.01.90 Bulletin 90/05**

(45) Publication of the grant of the patent:
**30.12.92 Bulletin 92/53**

(84) Designated Contracting States:
**CH DE FR GB IT LI**

(56) References cited:

**JOURNAL OF BIOTECHNOLOGY, vol. 8, no. 2, 8th June 1988, pages 123-134, Elsevier Science Publishers B.V., Amsterdam, NL; A. NAKAYAMA et al.: "Efficient secretion of the authentic mature human growth hormone by Bacillus subtilis"**

**BIOL. CHEM. HOPPE-SEYLER, vol. 367, August 1986, pages 731-740, DE; C. BERGMANN et al.: "Chemical synthesis and expression of a gene coding for Hirudin, the trombin-specific inhibitor from the leech Hirudo medicinalis"**

(73) Proprietor: **The Agency of Industrial Science and Technology**
**3-1, Kasumigaseki 1-chome**
**Chiyoda-ku Tokyo-to(JP)**

(72) Inventor: **Furutani, Yoshio**
**1004-3, Nagae, Hayamamachi Miura-gun Kanagawa-ken(JP)**
Inventor: **Honjo, Masaru**
**226, Takashi Mobara-shi Chiba-ken(JP)**
Inventor: **Nakayama, Akira**
**2785-1 Mutsuno Mobara-shi Chiba-ken(JP)**
Inventor: **Kawamura, Kouichi**
**2191-4 Tougou Mobara-shi Chiba-ken(JP)**
Inventor: **Mita, Izumi**
**2785-1 Mutsuno Mobara-shi Chiba-ken(JP)**
Inventor: **Ando, Kazunori**
**2785-1 Mutsuno Mobara-shi Chiba-ken(JP)**

BIOTECHNOLOGY, vol. 6, January 1988, pages 72-77, New York, N.Y., US; G. Loison et al.: Expression and secretion in S. cerevisiae of biologically active leech hirudin

(74) Representative: **Paget, Hugh Charles Edward et al**
**MEWBURN ELLIS & CO. 2/3 Cursitor Street London EC4A 1BO(GB)**

**Description**

SUMMARY OF THE INVENTION

The present invention relates to a novel method for preparing a hirudin. The hirudin can be defined as a protease inhibitor having an antithrombin activity which is present in sialaden of a Hirudo medicinalis. In contrast to conventional antithrombic medicines, the hirudin of the present invention has a direct inhibitory function to thrombin.

For this reason, the hirudin is expected as a preventive or remedy for thrombosis.

In conventional techniques, the small amount of the hirudin is merely extracted directly from Hirudo medicinalis. A technique is already known in which the hirudin is produced in E. coli, but the amount of the accumulated hirudin therein is so small that the technique is not considered to be useful in the medical field.

In the present invention, the employment of genetic engineering permits producing the hirudin in dramatically large quantities.

BRIEF DESCRIPTION OF THE DRAWINGS

Fig. 1 is a view illustrating an assembly process of a heterologous protein expressing and secreting vector pNPA225 used in examples.

Fig. 2 is a view illustrating an assembly process of a hirudin secreting vector pNPH208 used in the examples.

Fig. 3 is a view illustrating a syntherized oligonucleotide for coding amino acids of from the N-terminal to the tenth amino acid of a hirudin.

Fig. 4 is a view illustrating an assembly process of a hybrid plasmid p3009 containing the base sequence for coding the hirudin.

Here, explaining symbols together in these drawings, "promoter" denotes the promoter region of a neutral protease gene, "SD" a ribosome binding region within a neutral protease gene, "pre" a region coding for a secretion signal of the neutral protease gene, "Δpro" an upstream region of propeptide of the neutral protease gene, "α-amylase" a DNA sequence coding for α-amylase, "H" a DNA sequence coding for the hirudin, "ΔH" an upstream region of the DNA sequence coding for the hirudin, and "Δprotease" a back portion of the neutral protease gene.

DETAILED DESCRIPTION OF THE INVENTION

A hirudin produced in the present invention directly acts on thrombin in blood, and therefore it can be used for the medical treatment of thrombosis.

The hirudin is a protease inhibitor having an antithrombin activity present in sialaden of a Hirudo medicinalis which is a kind of eukaryote. Furthermore, the hirudin does not comprise a single protein but a group of several kinds of molecules. For example, HVI (Reference Literature 1) and HV2 (Reference Literature 2) are known as part of the molecules constituting the hirudin, and primary structures of them have already been reported (Reference Literature and 2).

These primary structures have high homology, and common structural features of these hirudin molecules are that a tyrosine residue in the form of a sulfate monoester is present therein and that three intramolecular S-S bonds are present therein.

In general, thrombin is present as inactive type prothrombin in blood, and it is activated in compliance with the demand of an organism in order to convert fibrinogen into fibrin. In short, thrombin takes charge of an important biological mechanism in a blood coagulation system. If thrombin is pathologically activated in the whole blood of an organism, fibrin is deposited in a microvascular system, so that thrombin are formed therein. The thrombus can be defined as a mass of coagulated blood in the blood vessel, and the pathological phenomenon for forming such thrombin is called thrombosis. The thrombosis leads to stricture and obstruction of the blood vessels owing to the thrombin, so that in main organs such as a heart, a cerebrum and lungs, an ischemic pathology or infarct take place in order to bring about their functional disorders. In recent years, much attention is paid to the thrombosis, because the latter is concerned with the occurrence of an organ pathology, which is attributable to immunological disorder, such as nephritis or pneumonites, and is also concerned with a concomitant pathology at the time of the transplantation of an organ or substitute blood vesssel. In addition, attention is also paid to diffuse intravascualr coagulation syndrome (DIC) known as a specific pathological symptom in which thrombi often occur in a microvascular system. The report regarding DIC has been made in 1960, and at first, DIC was considered to be a rare syndrome. However, it has been apparent of late that DIC is never rare, and with regard to various clinical symptoms which have been regarded as bleeding at the end of various diseases or as organ pathologies without any sufficient inspection, it is getting definite that these clinical symptoms result from the DIC.

Nowadays, the thrombosis has an increasing inclination. It is known that the thrombus is often formed at a position on the internal wall of the blood vessel where an abnormal change, particularly sclerosis or inflammation is present, and such a pathology increases with age. Furthermore, the worldwide elongation of life is one cause of the increase in the thrombosis.

Clinical pathlogical examples of the thrombosis include stroke, myocardial infarction, deep venous thrombosis, obstruction of limb arteries, pulmonary thrombosis and retinal artery thrombosis. The total thrombosises in various organs are at the top of all the diseases in points of disease incidence rate and death cause. In the future, therefore, the clinical and pathlogical significance of the thrombosis will be more and more important.

As remedies of the thrombosis just described, there are known heparin which functions through antithrombin III, and antivitamin K which inhibits the biosynthesis of a vitamin K-dependent blood coagulation factor. In addition, as another thrombin inhibitor, a gabexate mesylate agent which is a proteolytic enzyme inhibitor is also known.

Heretofore well-known heparin is an antithrombin agent which is often used for the thrombosises including DIC. However, the function of heparin is to accelerate the coagulation inhibitory effect of antithrombin III, and therefore heparin is not considered to be effective for the thrombosises associated with DIC or nephrosis where the content of antithrombin III is low (Reference Literature 3). Moreover, the gabexate mesylate agent has an inhibitory effect on physiologically important enzymes such as plasmin, kallikrein and trypsin. Accordingly, when the gabexate mesylate agent is used, great caution is required.

Under such circumstances, the development of a novel preventive or remedy for the thrombosises including DIC is important in therapeutic and preventive medicines.

On the other hand, a hirudin has a directly inhibitory activity to thrombin, in contract with the conventional agents for the thrombosis. In particular, the hirudin has high functional specificity to thrombin and prethrombin 2 (dissociation constant = $0.8 \times 10^{-10}$) (Reference Literature 4), and it merely inhibits activator No. IV alone in addition to thrombin. That is, the hirudin never inhibits enzymes other than ones regarding blood coagulation. Moreover, the toxicity of the hirudin is extremely low and it is called non-antigenicity, and the hirudin having a biological activity is rapidly excreted from the kidney into urine (Reference Literature 5). Thus, in place of the conventional antithrombic agents, the hirudin can be used as a useful preventive or remedy for thrombosises including DIC.

Before a recombinant DNA technique had not been developed, the hirudin was directly extracted from Hirudo medicinalis. However, in order to obtain even the small amount of the hirudin in this manner, a great amount of fasting Hirudo medicinalis is necessary, and even when the crude hirudin is produced, an intricate purification step and much time are required. For example, even in the case that the crude hirudin having a purity of about 10% is prepared in which impure proteins derived from the Hirudo medicinalis are present, a homogenate of the Hirudo medicinalis that have fasted for 2 or 3 weeks is first subjected to hot water extraction, and afterward ethanol precipitation, acetone fractional precipitation, adsorption and desorption by the use of bentonite, and finally isoelectric point precipitation must be successively carried out. Furthermore, the preparation of the pure hirudin from this crude product requires ECTEOLA cellulose column chromatography, Sephadex CM C-25 column chromatography and gel filtration by the use of Sephadex G-25, and according to a report, in such a case, yield is less than 0.001% (Reference Literature 6). Since it is impossible to obtain the hirudin in quantities as described above, this ingredient cannot be utilized yet in practice as a medicine, and the therapeutic utilization of the hirudin which can be expected from excellent characteristics thereof has not been achieved at present. For this reason, it is strongly demanded to establish an efficient production method of the hirudin.

In the near future, the mass production of a heterologous gene product will be possible by using the recombinant DNA technique in which a heterologous gene is expressed in the host of a microorganism. The heterologous gene product from the microorganism can be defined as a protein which results from the expression of a gene not derived from this microorganism.

The production of a desired substance by the use of the recombinant DNA technique in which a microorganism is used as a host can be roughly divided into intracellular production and extrabacterial secretory production.

In the former production, the heterologous gene product can be efficiently obtained in bacteria, but the following problems are posed:

In the first place, the heterologous gene product obtained in bacteria is usually an insoluble mass called an inclusion body having no biological activity. In general, it is not difficult to recover such an inclusion body from the bacteria. However, in order to acquire the heterologous gene product having a natural higher-order structure and activity, the inclusion body must be made soluble by using a solubilizing agent such as urea, and afterward it is necessary that the inclusion body is returned to the heterologous gene product having the natural higher-order structure and activity. This process is not considered to be practical, since it is very intricate and its efficiency is low. For example, according to Schoner et al. (Reference Literature 7), the inclusion boby of bovine growth hormone formed in E. coli is partially solubilized in the presence of 5 moles of urea, but the amount of the solublized inclusion body is nothing but 10% of the total amount and the rest thereof remains in the state of pellets.

In the second place, it is difficult to obtain the natural heterologous gene product.

Usually, in the case that the heterologous gene is expressed by using a microorganism, it is necessary that there is an initiation codon (ATG) at 5'-terminus of the gene coding for a protein. In consequence, a fused protein is produced in which Met is attached to an N-termial. If such a fused protein having the attached Met, as a medicine, is administered to a human, the incidence of an antibody based on the fused protein is heightened, so that side effects such as the deterioration of medicine efficacy and anaphylaxis take place inconveniently. In fact, a human growth hormone (hGH) produced in E. coli was an Met-hGH in which Met was attached to the N-terminal and which had about the same effect as in a natural hGH. However, when the Met-hGH was administered to a human, it was observed that the incidence of an antibody was high (Reference Literature 8).

Thirdly, it is also known that when the heterologous gene product having S-S bonds is prepared in bacteria, the S-S bonds are not crosslinked accurately. For example, it is reported by J.M. Schoemarker (Reference Example 9) that when a bovine chymosin having three S-S bonds in one molecule is expressed with the aid of E. coli, the S-S bond is formed between the molecules.

Also with regard to the hirudin, there is a known prior art (Reference Literature 10) for producing it in E. coli, but in this case, it is reported in this literature that the accumulated hirudin in the bacteria only has an antithrombin activity corresponding to 0.2 mg/ℓ•A600. The reason why the amount of the accumulated hirudin is so small would be that there is accumulated, in E. coli, the inactive hirudin where the S-S bond which is essential for the expression of the antithrombin activity is not precisely crosslinked.

Although a process to form the hirudin having the high antithrombin activity is now demanded, the intrabacterial production method is undesirable for the above-mentioned reasons.

On the other hand, the extrabacterial secretory production method for the useful heterologous gene product involves various problems to be solved, but nevertheless this method is considered to be more suitable for the object of the present invention.

In general, a secretory protein is synthesized in bacteria as a precursor protein in which a secretion signal is attached to the N-terminal of a mature protein, and the secretion signal of the precursor protein is removed therefrom in the course of secretion, so that the mature protein having no secretion signal is secreted from the bacteria (Reference Literature 11). Here, the mature protein mentioned above can be defined as a secretion signal-free secretory protein.

Therefore, if the hirudin is expressed in bacteria as the precursor protein in which the secretion signal is present at the N-terminal of the hirudin, and is then secreted from the bacteria, the thus obtained hirudin can have an antithrombin activity, whereby the above-mentioned problems of the extracellular secretory production method can be solved. In addition, if the ligation manner of the secretion signal to the hirudin is successfully contrived, the secretion signal is cleaved off at a desirable site on the hirudin at the time of the secretion, and it can be expected to produce the hirudin having the same N terminal as in the hirudin derived from Hirudo medicinalis.

Now, the inventors of the present application have investigated methods of using bacteria of Bacillus genus as a host which have the feature of secreting the great amount of a secretory protein and which are not pathogenic and which are often used as industrial microorganisms for enzymes, amino acids, nucleic acid and the like. Particularly, with regard to Bacillus subtilis in the Bacillus genus, abundant data are present from viewpoints of genetics, biochemistry, molecular biology and applied microbiology. Thus, it is significant to establish a host-vector system of using the Bacillus subtilis host by which the heterologous gene product is formed in Bacillus subtilis and is then secreted from the bacteria.

Heretofore, there have been made reports regarding the secretion of β-lactamase from E. coli by utilizing α-amylase-gene of Bacillus amyloliquefaciens (Reference Literature 12), the secretion of nuclease from Staphylococcus aureus (Reference Literature 13), the secretion of protein A from Staphylococcus aureus (Reference Literature 14), the secretion of human-α-interferon (Reference Literature 15), the secretion of mouse-β-interferon by the utilization of an α-amylase-gene of Bacillus subtilis (Reference Literature 16), the secretion of protein A from Staphylococcus by the utilization of a neutral protease gene or an alkaline protease gene of Bacillus amyloliquefaciens (Reference Literature 17), and the secretion of human serum albumin (Reference Literature 18). According to these reports, when Bacillus subtilis is used as the host, a protein derived from a certain kind of procaryote can be secreted and accumulated efficiently.

For example, amounts of the secreted and accumulated $\beta$-lactamase (Reference Literature 12), nuclease (Reference Literature 13) and protein A (Reference Literature 17) are about 20 mg, about 50 mg and about 1,000 mg, respectively, per liter of a culture medium.

However, the above-mentioned reports indicate that the secreted levels of human-$\alpha$-interferon (Reference Literature 15), mouse-$\beta$-interferon (Reference Literature 16), human serum albumin (Reference Literature 18) derived from eukaryotes are very low. For example, there is the report that the amount of the human-$\alpha$-interferon is 0.5 mg per liter of a culture medium. As exhibited in these reports, it is not always easy to obtain the large amount of a protein derived from an eukaryote by the use of Bacillus subtilis as the host.

In general, the gene of the secreted protein is transcribed into mRNA by RNA polymerase, and this mRNA is used as a template in order to synthesize a precursor protein in which a secretion signal is added to the N-terminal of a mature protein. It is known that the secretion signal is removed from the precursor protein in the course of the secretion.

For the purpose of inspecting a cause why the amount of the secreted and accumulated protein depends upon the kind of protein itself, Ulmanen et al. (Reference Literature 19) have investigated the transcription and translation of a fused gene in which a DNA sequence coding for a heteroprotein is ligated to a site immediately preceding the region coding for a promoter, ribosome binding region and secretion signal of a secretion-protein-gene, and further have investigated a secretion efficiency of the resulting heterologous gene product, in order to ascertain what of the above-mentioned factors has an influence on the amount of the secreted and accumulated heterologous gene product.

That is, they have tested the secretion of EI protein which is the glycoprotein of Semiliki Forest virus, $\beta$-lactamase of E. coli and $\alpha$-amylase-gene of Bacillus amyloliquefaciens by the use of the region for coding a promoter, ribosome binding region and secretion signal of the amylase-gene of Bacillus amyloliquefaciens. As a result, it has been apparent that the amount of the mRNA coding for the precursor protein, in which the secretion signal is added to the site above the N-terminal of a mature protein produced in the bacteria, scarecely changes.

On the other hand, it has been reported that amounts of secreted and accumulated E1 prtein and $\beta$-lactamase are 0.01% and 10%, respectively, of the amount of the secreted and accumulated $\alpha$-amylase. According to this report, the cause of the above-mentioned results is that Bacillus subtilis cannot efficiently secrete certain kinds of fused protein in which a DNA sequence for heterologous protein is fused to the 3'-terminal of the secretion signal coding region of the $\alpha$-amylase gene, or the cause is due to the degradation of the heterologous protein by a host proteolytic activity

after or during secretion. As understood from the foregoing, it has been reported that even if the same secretion protein gene, i.e., the same region coding for the promoter, ribosome binding region and secretion signal of the $\alpha$-amylase-gene is used, the amount of the secreted and accumulated protein depends upon a kind of protein itself.

According to the report of Palva. I (Reference Literature 15) and Schein C.H. (Reference Literature 20), even if the same region coding for the secretion signal of a secretory protein gene and the same DNA fragment coding for a protein derived from an eukaryote are used, a difference of a ligation structure arises between both of them, so that in one case, the protein derived from the eukaryote is secreted by the Bacillus subtilis, or in another case, it is not secreted thereby.

Palva et al. have reported the following experiment: A DNA fragment coding for a mature interferon (IFN) is ligated, directly or via a junction region (Asn-Gly-Thr-Glu-Ala) comprising 5 amino acid residues, to a site below the region (Ala-Val) containing a cleavage site of the secretion signal in $\alpha$-amylase-gene of Bacillus amyloliquefaciens which is one of secretion proteins, i.e., a DNA fragment containing the region (Val) coding for an N-terminal amino acid of a mature protein on the downstream side of the 3'-terminal coding for the secretion signal. In this case, the secretion signal is removed, but the secreted and accumulated interferon is a fused protein in which one amino acid (Val) is added to the N-terminal of the mature interferon, or it is another fused protein in which 6 amino acids (Val-Asn-Gly-Thr-Gln-Ala) are added thereto, and the total amount of these interferons is from 0.5 mg to 1 mg per liter of a culture medium. On the other hand, Schein et al. have tried the secretion of a human IFN by ligating the DNA fragment coding for the mature interferon (IFN) to a site immediately after the region coding for the amino acid (Ala) of the C-terminal of the secretion signal of the same $\alpha$-amylase-gene as in Palva et al. In this case, however, it has been reported that a great amount of a precursor IFN or the mature IFN remains in the cells of the bacteria and the efficiency of the secretion into a culture medium is very low.

6

As discussed above, it is unpredictable whether or not the precursor in which the secretion signal is fused to a site above the N-terminal of the mature protein derived from an eukaryote is secreted into a system of using the Bacillus subtilis as the host, and whether or not the secretion signal is processed in the course of the secretion. It is widely recognized that these results depend upon the combination of a selected mature protein and secretion signal, or the ligation manner of both of them.

Under such circumstances, the present invention has been achieved, and an object of the present invention is to provide a hirudin which is a useful antithrombinic agent as a preventive or remedy for thrombosis, and other objects of the present case are to provide a hirudin secretion plasmid, to provide a transformed strain, and to provide a method for preparing the hirudin by the use of this transformed strain.

Taking the above-mentioned situations into consideration, the present inventors have made investigations to search a gene having a promoter, ribosome binding region and secretion signal which permit secreting a hirudin, and as a result, the present invention has been finally completed.

That is, the present invention is directed to a hirudin secretion plasmid in which a DNA sequence is ligated to a vector DNA, the aforesaid DNA sequence being prepared by ligating a DNA fragment coding for the hirudin to a site immediately after the region coding for the promoter, ribosome binding region and secretion signal of the neutral protease gene of Bacillus amyloliquefaciens; a transformed strain obtained by transforming with the aforesaid hirudin secretion plasmid; and a method for preparing a hirudin which comprises the steps of culturing the aforesaid transformed strain, and recovering the hirudin from the supernatant of a culture medium.

Now, the present invention will be described in detail.

The promoter referred to in the present invention can be defined as a DNA sequence which is recognized by a RNA polymerase and to which the latter is bonded.

It is known that when the initiation point of RNA synthesis is regarded as "+1", two consensus DNA sequences are usually present at -35 region (5'-TTGACA-3') and -10 region (5'-TATAAT-3'). In general, it is considered that the "-35 region" is necessary for the recognition of the RNA polymerase and the "-10 region" is necessary for the ligation of the RNA polymerase (Reference Literature 21).

As already known, Bacillus subtilis has various kinds of RNA polymerases. This versatility plays important roles in the course of spore formation involving the intricate expression control of the Bacillus subtilis. In particular, most polymerases in a vegatative propagation phase include ass type RNA polymerase, and therefore, it is known that the transcription of most genes is carried out by the aid of this type of polymerase (Reference Literature 22).

The DNA sequence of the present invention, in which the DNA fragment coding for the hirudin is ligated to a site immediately after the region coding for the promoter, ribosome binding region and secretion signal of the neutral protease gene of Bacillus amyloliquefaciens, is as follows:

7

```
5'GATCTTAACATTTTTCCCC

TATCATTTTTCCCGTCTTCATT

TGTCATTTTTTCCAGAAAAAAT

CGTCATTCGACTCATGTCTAAT

CCAACACGTCTCTCTCGGCTTA

TCCCCTGACACCGCCCGCCGAC

AGCCCGCATGGACGAATCTATC

AATTCAGCCGCGGAGTCTAGTT

TTATA(TTGCAG)ᵃAATGCGAG

ATTGCTGGT(TTATTAT)ᵇAAC

AATATAAGTTTTCATTATTTTC

AA(AAAGGGG)ᶜGATTTATT(G

TGGGTTTAGGTAAGAAATTGTC

TAGTGCTGTAGCCGCTTCCTTT

ATGAGTTTAACCATCAGTCTGC

CGGGTGTTCAGGCC)ᵈ(GTTGT

TTATACAGATTGCACAGAATCC

GGACAAAATTTATGTTTATGTG

AAGAATCTAATGTTTGTGGACA

AGGAAATAAATGTATTTTAGGA

TCTGATGGAGAAAAAAATCAAT

GTGTTACAGGAGAAGGAACACC

GAAACCGCAATCTCATAATGAT

GGAGATTTTGAAGAAATTCCTG

AAGAATATTTACAA3')ᵉ
```

In this DNA sequence, the "-10 region" of the promoter is considered to be 5'TATTAT3' (b portion in the above sequence) and the "-35 region" is considered to be 5'TTGCAG3' (a portion in the above sequence).

This DNA sequence has a high homology to the consensus sequence recognized by the σ-55type RNA polymerase which is the main RNA polymerase in the vegetative propagation phase of Bacillus subtilis -

(Reference Literature 22).

Furthermore, the ribosome binding region can be defined as the DNA sequence encoding a region of mRNA to which ribosome can be bound mRNA synthesized by the RNA polymerase combines with a ribosome.

In general, the ribosome binding region is DNA sequence which is present 5 to 9 base upstream of the initiation codon which is complementary to 3'-terminal sequence of the 16S RNA an initiation codon and which is complementary to that of a 16S rRNA3' terminal. The DNA sequence of the 16S rRNA depends upon a kind of microorganism, but it is known that the DNA sequence of the 16S rRNA of Bacillus subtilis is 3'UCUUUCCUCC5' (Reference Literature 22).

As the DNA sequence which is considered to be the ribosome binding region in the above-mentioned DNA sequence, there is a sequence of 5'AAAGGGG3' (c portion in the above shown sequence), and this DNA sequence has an extremely high complementarity to the 16S rRNA of Bacillus subtilis.

The DNA sequences of the promoter and ribosome binding region just described play an important role for the expression of the gene. Nowadays, it is widely known that these DNA sequences are concerned with the expression efficiency of a gene (Reference Literature 21).

In the case that the gene of a desired protein is expressed by using a Bacillus bacterium as a host, the RNA polymerase and ribosome of the Bacillus bacterium have a strict specificity to the promoter and ribosome binding region (Reference Literature 22), and therefore the regions thereof are desirably derived from the Bacillus bacterium (Reference Literature 23).

The secretion signal can be defined as a polypeptide comprising 20 to 30 amino acid residues present preceding the N-terminal of a mature protein. Characteristics of the secretion signal are as follows: It is known that a basic amino acid is present near the N-terminal, that the cluster of a hydrophobic amino acid exists in the middle thereof, and that an amino acid having a small side chain is present at a cleavage site of the secretion signal. This polypeptide will be removed in the course of the secretion and is considered to play an important role in passing through a cytoplasmic membrane of a precursor protein (Reference literature 11).

The amino acid sequence which is considered to be the secretion signal of the neutral protease gene of Bacillus amyloliquefaciens used in the assembly of the hirudin secretion plasmid according to the present invention is

```
Met-Gly-Leu-Gly-Lys-Lys-Leu-Ser-Ser-Ala-Val-Ala-Ala-Ser-Phe-

Met-Ser-Leu-Thr-Ile-Ser-Leu-Pro-Gly-Val-Gln-Ala-,
```

and this sequence has a typical primary structure of the secretion signal. That is, in this amino acid sequence, it can be perceived that Lys which is a basic amino acid is present in the vicinity of the N-terminal, that the cluster

```
                                                    (Lue-Ser-

ser-Ala-Val-Ala-Ala-Ser-Phe-Met-Ser-Leu-Thr-Ile-Ser-Leu-)
```

of a hydrophobic amino acid exists in the middle thereof, and that an amino acid (Ala) having a small side chain is present at a cleavage site of the secretion signal.

Thus, the DNA sequence coding for the secretion signal in the above-mentioned DNA sequence is

5'GTGGGTTTAGGTAAGAAATT

GTCTAGTGCTGTAGCCGCTTCC

TTTATGAGTTTAACCATCAGTC

TGCCGGGTGTTCAGGCC3'

(d portion of the above-mentioned DNA sequence).

The amino acid sequence of the hirudin protein used to assemble the hirudin secretion plasmid of the present invention is

Val-Val-Tyr-Thr-Asp-Cys-Thr-Glu-Ser-Gly-Gln-Asn

-Leu-Cys-Leu-Cys-Glu-Gly-Ser-Asn-Val-Cys-Gly-Gln

-Gly-Asn-Lys-Cys-Ile-Leu-Gly-Ser-Asp-Gly-Glu-Lys

-Asn-Gln-Cys-Val-Thr-Gly-Glu-Gly-Thr-Pro-Lys-Pro

-Gln-Ser-His-Asn-Asp-Gly-Asp-Phe-Glu-Glu-Ile-Pro

-Glu-Glu-Tyr-Leu-Gln,

and the DNA sequence coding for this hirudin is

5'GTTGTTTATACAGATTGCA

CAGAATCCGGACAAAATTTATG

TTTATGTGAAGAATCTAATGTT

TGTGGACAAGGAAATAAATGTA

TTTTAGGATCTGATGGAGAAAA

AAATCAATGTGTTACAGGAGAA

GGAACACCGAAACCGCAATCTC

ATAATGATGGAGATTTTGAAGA

AATTCCTGAAGAATATTTACAA3'

(e portion of the above-mentioned DNA sequence).

In recent years, DNA sequences of many genes have been elucidated, and it is possible to inspect the frequency of codons which are utilized in the genes. As a result, it has become apparent that the frequency of the utilized codons depends upon a kind of creature. Thus, in the case that the DNA sequence is chemically synthesized so as to obtain a high efficient expression, it is the usual way to design the DNA sequence so that suitable codons may be sufficiently contained therein. In assembling the hirudin secretion plasmid of the present invention, the DNA sequence coding for chemically synthesized hirudin is used. Usually, a DNA sequence coding for a polypeptide is not limited to one kind of codon, and therefore the several DNA sequences coding for hirudin can be conceived. However, in the present invention, the DNA sequence which is designed so as to contain many desirable codons for Bacillus subtilis is employed, taking it into consideration that the expression is made using the Bacillus subtilis as a host.

For the secretion of heterologous protein by Bacillus subtilis, it is essential that the hetrologous gene coding for the desired protein is ligated to the region coding for the promoter, ribosome binding region and secretion signal which is derived from a gene for extracellular protein. It is important to use the gene for the extracellular protein which can be produce extracellularly in a large amount in a short period of time.

As such secretion proteins, alkaline protease, α-amylase, levan sucrase and the like are known. However, it should be noted that the heterologous gene product cannot be efficiently secreted at times only by assembling the heterologous protein secretion plasmid in which the DNA fragment coding for the heterologous protein is ligated to a site following the region coding for the promoter, ribosome binding region and secretion signal of such a secretion protein and which can be replicated with the Bacillus subtilis.

This fact has been reported by Ulmanen et al. (Reference Literature 19) and Schein (Reference Literature 20).

The inventors of the present application have intensively researched to obtain the gene coding for the secretion protein having the ability by which hirudin can be secreted in large quantities from the bacteria, and they have also investigated a ligation manner between the DNA fragment coding for the secretion signal and the DNA fragment coding for the hirudin. As a result, it has been found that when the neutral protease gene of a Bacillus bacterium, particularly preferably Bacillus amyloliquefaciens is selected, and when the DNA fragment coding for the hirudin is ligated to a site immediately after the region coding for the promoter, ribosome binding region and secretion signal, it is possible to assemble the secretion plasmid of the present invention having such extremely excellent results as shown in examples of the present case.

As a vector DNA for constituting the hirudin secretion plamid of the present invention, any one can be used, so long as it can be replicated in Bacillus substilis. Examples of the vector which can usually often be used include pUB110, pTP5, pC194, pDB9, pBD64, pBC16 and pE194 which are plasmids derived from Staphylococcus. The Bacillus subtilis having the above-mentioned plasmids can be available to everyone in Bacillus Stock Center in Ohio University (address: 484 West 12th Avenue, Columus, Ohio 43210 USA).

In particular, as the vector DNA used in the present invention, any one can be utilized, so long as it is a plasmid which can be replicated by Bacillus bacteria. However, pUB110 is best, since data of molecular biology about it are plentiful, and it can be retained stably in the Bacillus bacteria.

As described in examples given hereinafter, the hirudin secretion plasmid of the present invention can be prepared by ligating the DNA fragment coding for the hirudin to a site immediately after the region coding for the promoter, ribosome binding region and secretion signal of a neutral protease gene by the use of a heterologous protein expression vector which is capable of coding a heterologous protein immediately after the region coding for the above promoter, ribosome binding region and secretion signal. The hirudin secretion plasmid of the present invention can be assembled by combining a chemically synthesized DNA fragment containing the above-mentioned DNA sequence with a vector DNA fragment cleaved with a suitable restriction enzyme which can be replicated in Bacillus subtilis in accordance with a usual ligation technique. In these cases, the two DNA fragments can be ligated to each other, for example, via a common restriction enzyme site, and/or by using a synthesized DNA linker, and/or by means of a blunt end ligation.

The Bacillus subtilis can be transformed to obtain a transformed strain by the use of the hirudin secretion plasmid assembled by ligating the DNA fragment to the above-mentioned vector DNA, the aforesaid DNA fragment being prepared by ligating the DNA fragment coding for the hirudin protein to a site after the region coding for the promoter, ribosome binding region and secretion signal of the neutral protease gene of the Bacillus amyloliquefaciens.

As a technique for transforming the Bacillus subtilis, any one can be employed, so long as it is a technique which is now used in the art.

For example, a method suggested by Chang et al. (Reference Literature 24) can be used. This method can be divided into three steps as follows:

(1) The step of producing cell wall-free Bacillus subtilis, i.e., a protoplast by treating Bacillus subtilis with lysozyme in an isotonic solution.

(2) The step of transforming the protoplast with a vector DNA in a polyethylene glycol solution.

(3) The step of reproducing the cell wall for the protoplast in a reproductive culture medium, and selecting the transformed Bacillus subtilis.

In order to obtain the hirudin by using the thus prepared transformed strain, the liquid cultivation of the strain is carried out by the usual method. For example, the transformed strain is planted in 400 ml of a LB culture medium (Reference Literature 25) in a 2-liter Erlenmeyer flask, and cultivation is then performed at 37°C for about 20 hours with stirring, preferably until a maximum yield of the hirudin has been secreted and produced.

The transformed strain of the present invention can be cultivated in a liquid culture medium containing a digestible carbon source, nitrogen source and inorganic salts source. For example, the liquid culture medium which can usually often be used is a LB culture medium.

As the strain used in the present invention, any one can be employed, so long as it is a Bacillus bacterium which can be transformed by the hirudin secretion plasmid of the present invention. Bacillus subtilis is best, because data based on genetics, biochemistry, molecular biological and applied microbiology are plentiful, and safety is high.

The preparation of the hirudin from the culture medium can be achieved by the recovery purification from the supernatant of the culture medium. The procedure of the hirudin acquisition found by the present inventors is as follows: The medium supernatant is regulated to a pH of 3 with hydrochloric acid, and treatment is then carried out at 70°C for 15 minutes to form a protein precipitate. Afterward, the latter is removed therefrom by centrifuging to obtain a supernatant fraction containing hirudin. In the supernatant fraction, the hirudin is present in a high ratio of 20% with respect to the total protein and only contains the very small amount of impure proteins. Therefore, the secreted hirudin in the supernatant of the culture medium can be easily purified by a cation exchange chromatography, an anion exchange chromatography and an affinity chromatography.

The present inventors have found that the hirudin having an antithrombin activity corresponding to 10 mg/ℓ·A660 can be secreted and accumulated by cultivating, in a 2-fold concentrated LB culture medium, Bacillus subtilis transformed with the hirudin secretion plasmid. In this case, the absorbance (A660) of the culture medium which denotes the growth degree of the Bacillus subtilis is 10. The above-mentioned unit (mg/ℓ·A660) shows a value obtained by dividing the amount (mg) of the accumulated hirudin in the medium supernatant (1 liter) by the absorbance (A660) indicating the growth degree of the Bacillus subtilis in the culture medium.

It has been found that 35 mg of the hirudin can be obtained from 1 liter of the culture medium by regulating this supernatant (1 liter) to a pH of 3 with hydrochloric acid, then treating it at 70°C for 15 minutes to form a protein precipitate, removing the latter therefrom by centrifuging to obtain a supernatant fraction, and purifying the supernatant fraction by the use of an anion exchange chromatography and an affinity chromatography. The present invention has been achieved on the basis of this knowledge.

Furthermore, the hirudin secretion plasmid of the present invention has the DNA region in which the 5' terminal of the DNA fragment of the hirudin is directly ligated to a site immediately below the 3' terminal of the secretion signal region of the neutral protease gene, and it can be considered that in the bacteria or cytoplasmic membranes of the transformed strain obtained by performing transformation with this plasmid, a precursor protein is synthesized in which an amino acid present at the N-terminal of the hirudin is bound to a site immediately after an amino acid present at the C-terminal of the neutral protease secretion signal. It is known that the secretion signal is generally removed in the process of the secretion, and also in the present invention, there can be expected the secretion and production of such a natural type hirudin having the N-terminal amino acid sequence as in a secretion signal-free hirudin derived from Hirudo medicinalis. According to the report of Schein et al. (Reference Literature 20), however, only by ligating the DNA fragment coding for a desired mature protein to a site immediately after the region coding for the promoter, ribosome junction region and serection signal of the gene coding for a secretion protein, the mature protein from which the seretion signal has been removed cannot be efficiently secreted at times from the precursor protein which is considered to be synthesized in bacteria and in which the mature protein is fused to the C-terminus of the secretion signal. Also in the case of the Bacillus subtilis transformed with the hirudin secretion plasmid in the present invention, it has been indefinite whether or not the natural hirudin from which the secretion signal has been removed is secreted from the precursor protein in which an amino acid present at the N-terminal of hirudin is bound to a site immediately after an amino acid present at the C-terminal of the neutral protease secretion signal.

The present inventors have investigated various ligation manners between the secretion signal and hirudin, and as a result, they have found that only by the ligation manner of the present invention, the natural type hirudin is secreted into a culture medium with the aid of the transformed Bacillus subtilis, as shonw in examples of the present case.

As shown in one embodiment of the present invention, the hirudin having the same N-terminal amino acid sequence and the same amino acid composition as in the hirudin derived from Hirudo medicinalis can be secreted into the supernatant of a culture medium without any disintegration by utilizing the promoter, ribosome binding region and secretion signal region of the neutral protease gene of Bacillus amyloliquefaciens to assemble a hirudin secretion plasmid, then introducing this plasmid into Bacillus subtilis to obtain a transformed strain, and cultivating the strain. That is, a hirudin preparation method has been established by which the hirudin can be easily recovered and purified.

Now, the present invention will be described in detail in reference to examples, but the scope of the present invention should not be limited by these examples.

Example 1

(Assembly of Heteroprotein Expressing and Secreting Vector pNPA225)

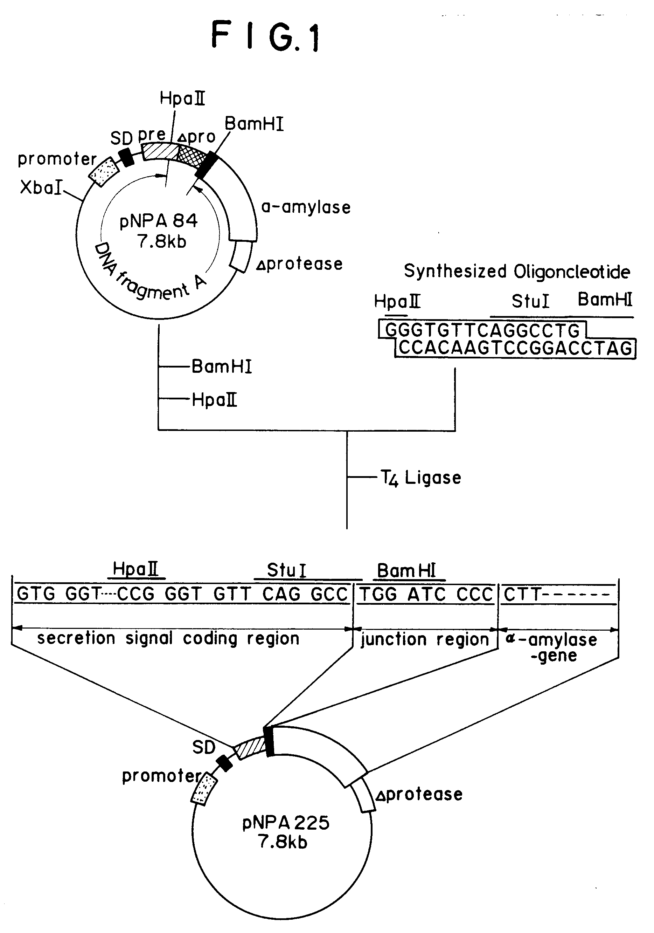

A method shown in Fig. 1 was performed to assemble a heterologous protein expressing and secreting vector pNPA225 in which the DNA fragment coding for the heterologous Protein was ligated to a site immediately after the region coding for the promoter, ribosome binding region and secretion signal of the neutral protease gene of Bacillus amyloliquefaciens.

In Fig. 1, the plasmid pNPA84 is an amylase secretion plasmid having the DNA fragment in which the DNA fragment (α-amylase) coding for the mature α-amylase protein is ligated to the site after the region coding for a promoter (promoter), ribosome binding region (SD), secretion signal (pre) and propeptide (Δpro) of the neutral protease gene. The mature α-amylase gene can be defined as the DNA fragment coding for a naturally occurring protein having an α-amylase activity and beginning with leucine. From the transformed strain MT-8400 (FERM BP-923) containing this plasmid pNPA84, the latter pNPA84 was prepared in accordance with the method of Tabak et al. (Reference Literature 26). This pNPA84 DNA was then cleaved with restriction enzymes HpaII and BamHI (which were both made by Takara Shuzo Co., Ltd.) in order to obtain about 7.8 Kb of a DNA fragment (hereinafter referred to as "DNA fragment A"), and the latter was further purified by means of an electrophoresis using agarose gel. This DNA fragment contained the region coding for the promoter, ribosome binding region and C-terminal-free secretion signal. On the other hand, for the purpose of cleaving the neutral protease gene immediately after the secretion signal region thereof with a restriction enzyme StuI, two kinds of oligonucleotides, i.e., 16mer and 18mer were synthesized by an improved triester method (Reference Literature 27). Afterward, 1 μg of each of two kinds of synthesized oligonucleotides was phosphorylated by the use of T4 polynucleotide kinase (made by Takara Shuzo Co., Ltd.) and dATP (made by Pharmachia Labs., Inc.) (Reference Literature 28). Next, these reaction products were mixed and then heated for 3 minutes in hot water, followed by slow cooling, thereby annealing the two kinds of synthesized oligonucleotides. Afterward, the DNA fragment (0.5 μg) and the annealed synthesized oligonucleotide (1 μg) were ligated to each other by the use of a T4 ligase (made by Takara Shuzo Co., Ltd.) in order to obtain a hybrid plasmid pNPA 225.

Example 2

(Assembly of Hirudin Secreting Vector pNPH208)

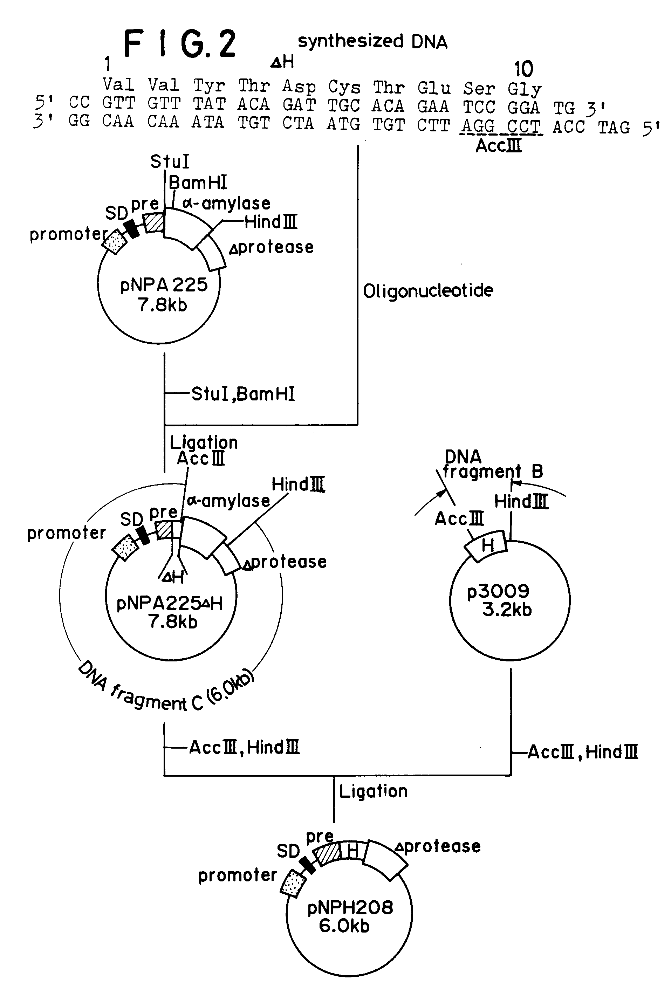

A plasmid pNPH208 was assembled in accordance with the method shown in Fig. 2.

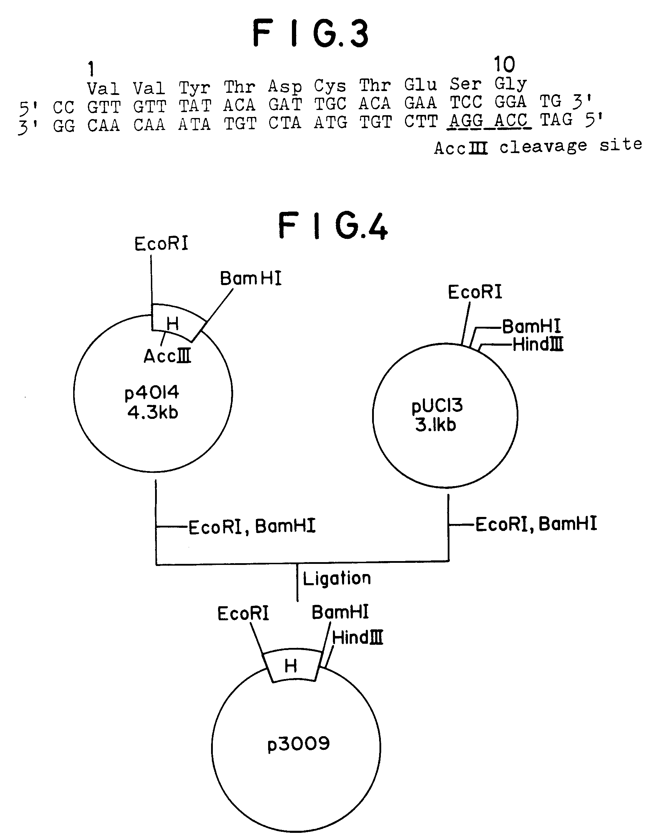

In the first place, for the purpose of assembling a DNA fragment (Fig. 3) containing the DNA region coding for an amino acid of from the N-terminal to the 10th residue of a hirudin, two kinds of oligonucleotides were synthesized. Next, 1 μg of each of two kinds of synthesized oligonucleotides was phosphorylated and then annealed. This was ligated to pNPA225 DNA (0.5 μm), which had been cleaved with restriction enzymes StuI and BamHI, by the use of a T4 ligase (made by Takara Shuzo Co., Ltd.) in order to obtain a hybrid plasmid pNPA225ΔH in which the N-terminal region (which corresponded to the portion of from the N-terminal to the 10th amino acid residue of the hirudin) of the DNA fragment coding for hirudin was ligated to a site immediately after the region coding for the secretion signal of the neutral protease gene.

There is already deposited a strain (FERM BP-1985) which is transformed by the plasmid p4014 assembled from a vector pBR322 DNA and a synthesized DNA prepared by selecting a codon corresponding to the amino acid sequence of the hirudin from codons often used in Bacillus subtilis, i.e., optimum codons (Reference Literature 29), and then chemically synthesizing the codon. The DNA fragment (H) coding for the hirudin was produced from this transformed strain B9-1985 in the following manner: First, p4014 was cleaved with restriction enzymes EcoRI and BamHI to prepare a DNA fragment containing the

13

hirudin. The thus prepared DNA fragment was arranged between cleavage sites of plasmid pUC 13 with the restriction enzymes EcoRI and BamHI to assemble a hydrid plasmid p3009 (Fig. 4). This p3009 DNA (2 μg) was cleaved with the restriction enzymes AccIII and HindIII to prepare the DNA fragment (DNA fragment B) containing the DNA region coding for the amino acid sequence of from the eleventh amino acid to the C-terminal of the hirudin, and the DNA fragment B was then purified by means of electrophresis.

This DNA fragment B and pNPA 225 ΔH were cleaved with restriction enzymes AccIII and HindIII to produce about 6.0 Kb of a DNA fragment (DNA fragment C) (1 μg) was ligated by the use of T4 ligase, thereby assemble a hirudin secretion plamid pNPH 208. This pNPH 208 was a hybrid plasmid containing the DNA sequence in which the DNA fragment coding for the hirudin was ligated to a site immediately below the region coding for the promoter, ribosome binding region and secretion signal of the neutral protease gene of Bacillus amyloliquefaciens.

Example 3

(Secretion and Productin of Hirudin by Hirudin Secretion Plasmid)

By the use of the plasmid pNPH208 assembled in Example 2, a Bacillus subtilis MT-430 strain (FERM BP-1079) was transformed in accordance with the method of Chang et al. (Reference Literature 24). The resulting transformed strain MT-208 (FERM BP-1983) was subjected to a shake culture process at 37°C for 20 hours by the use of a 2-fold concentrated LB culture medium. The amount of a secreted and accumulated hirudin was determined by measuring an antithrombin activity in the supernatant of the culture medium. This antithrombin activity was determined by measuring an inhibitory degree of the hydrolysis activity of a synthesized substrate H-D-phenylanil-L-pipecolyl-L-arginyl-p-nitroanilide to thrombin (Reference Literabute 30). That is, 50 μℓ of a thrombin (made by Mochida Pharmaceutical Co., Ltd.) solution (20 IU/ml) was mixed with 50 μℓ of a buffer solution (50 mM Tris-HCl, pH = 8.0) under neutral to acidic conditions, and the mixture was then incubated at room temperature for 2 minutes. Afterward, 50 μℓ of the mixture was added to H-D-phenylanil-L-pipecolyl-L-arginyl-p-nitroanilide (Daiichi Chemical Pharmaceutical Co., Ltd.) (final concentration of the substrate = 0.25 mM). After the commencement of reaction, the liberation of p-nitroanilide was measured at a wavelength of 405 nm, and the increment of absorption per unit time was represented by a. Next, the buffer solution was replaced with a sample solution, and the similar procedure was then carried out in order to measure the increment of absorption at a wavelength of 405 nm. The thus measured value was represented by b. An antithrombin activity of the supernatant of the culture medium was determined by calculating (a-b)/a. One unit of the antithrombin activity can be defined as an ability to neutralize one unit of 1N thrombin. The content of the hirudin was determined by first separately measuring a thrombin specific activity and then utilizing the fact that the hirudin inhibits thrombin in a ratio of 1:1 (molar ratio).

After cultivation had been carried out at 37°C for 20 hours by the use of a 2-fold concentrated LB culture medium, an antithrombin activity of about 116 million units, i.e., about 100 mg of the accumulated hirudin per liter of the culture medium was observed. In this case, the absorbance (A660) of the culture medium which denoted the growth degree of the Bacillus subtilis was 10. Furthermore, the antithrombin activity of the secreted and accumulated hirudin did not decrease but increased with time. When a precipitate obtained by adding trichloroacetic acid to the supernatant of the culture medium was analyzed with SDS-PAGE, it became apparent that a protein having the same size as a hirudin preparation obtained by purifing a hirudin commercially sold from Sigma Chemical Co. was accumulated in large quantities in the supernatant of the culture medium. In addition, according to analytical results by a gel scanner densitometer, the amount of the hirudin in the supernatant of an MT-208 strain culture medium was as much as 5%. When this supernatant was thermally treated at 70°C for 15 minutes to form a proteinous precipitate and the latter was then removed therefrom by means of centrifuging, the antithrombin activity was still present in the supernatant fraction. Moreover, the ratio of the existing hirudin protein to the total protein was heightened up to 20%, by which it was meant that impure proteins were remarkably decreased. The hirudin (Reference Literature 31) obtained by purifying this supernatant showed one band in SDS-PAGE, and it became apparent that the molecular weight of this hirudin was the same as that of a hirudin preparation obtained by purifing a hirudin derived from Hirudo medicinalis commercially available from Sigma Chemical Co. The N-terminal amino acid sequence of the purified hirudin was then measured, and as a result, it was determined that the sequence was Val-Val-Tyr-Thr-Asn.

The purified hirudin (10 μg) was hydrolyzed at 110°C for 24 hours in 6N hydrochloric acid, and the hydrolyzed substance was then analyzed for an amino acid composition (Reference Literature 33). The results are set forth in Table 1. It is definite that the analytical values in the table are closely in accord with theoretical values of an amino acid composition calculated from the amino acid sequence of a hirudin derived from Hirudo medicinalis.

This fact indicates that the hirudin which has been secreted and produced from the Bacillus subtilis transformed with the hirudin secretion plasmid of the present invention has the same N-terminal amino acid sequence and the same amino acid composition as in the hirudin derived from Hirudo medicinalis.

As a result of a thrombin inhibition experiment, it became apparent that this purified hirudin was capable of reacting with thrombin stoichiometrically in a ratio of 1:1.14.

This fact also indicates that the hirudin which has been secreted and produced from the Bacillus subtilis transformed with the hirudin secretion plasmid of the present invention has the same antithrombin activity as in the hirudin derived from Hirudo medicinalis.

Table 1

| Analytical Results of Amino Acid Composition | | | |
|---|---|---|---|
|  | Number of Amino Acid | Theoretical Value | Found Value |
| Asp + Asn | 9 | 15.25 | 14.98 |
| Thr | 4 | 6.78 | 6.58 |
| Ser | 4 | 6.78 | 6.29 |
| Glu + Gln | 13 | 22.03 | 22.74 |
| Pro | 3 | 5.08 | 6.32 |
| Gly | 9 | 15.25 | 16.03 |
| Ala | 0 | 0 | 0.5 |
| Val | 4 | 6.78 | 5.67 |
| Met | 0 | 0 | 0.0 |
| Ile | 2 | 3.39 | 3.32 |
| Leu | 4 | 6.78 | 6.54 |
| Tyr | 2 | 3.39 | 2.69 |
| Phe | 1 | 1.69 | 1.66 |
| His | 1 | 1.69 | 1.71 |
| Lys | 3 | 5.08 | 5.01 |
| Arg | 0 | 0 | 0.0 |
|  | (59) | 100 | 100 |

List of Literature

1. Dodt, J. et al., FEBS Lett., 165, 180-184 (1984)

2. Harvery, R.P. et al., Proc. Natl. Acad. Sci. USA, 83, 1084-1088 (1986)

3. Rosenberg, R.D., Semin. Hematol., 14427-440 (1977)

4. Markwardt, F., "Methods in Enzymol.", 19, 924-932 (1970)

5. Markwardt, F. et al., Thromb. Haemostasis (Stuffgart), 52, 160-163 (1984)

6. Badgy, D. et al., "Methods in Enzymol.", 45, 669-678 (1976)

7. Schoner, R.G. et al., BIO/TECHNOLOGY, 3, 151-154 (1985)

8. Naomi Hizuka, BIO medica, 2(1), 79-83 (1987)

9. J.M. Schoemaker et al., ENBO J., 4, 775 (1985)

10. Japanese Patent Unexamined Publication No. 61-501609

11. Blobel, G. et al., Cell. Biol., 67, 835 (1975)

12. Palva, I. et al., Proc. Natl. Acad. Sci. USA, 79, 5582-5586 (1982)

13. Kovacevic, S. et al., J. Bacteriol., 162, 521-528 (1985)

14. Fahnestock, S.R. et al., J. Bacteriol., 165, 796-804 (1986)

15. Palva, I. et al., Gene, 22, 229-235 (1983)

16. Yamane, K. et al., In J.A. Hoch and P. Setlow (ed.)., Molecular Biology of Microbial Differentiation, American Society for Microbiology, Washington, D.C., 117-123 (1985)

17. Vasantha, N. et al., J. Bacteriol., 165, 837-842 (1986)

18. Saunders, C.W. et al., J. Bacteriol., 169, 2917-2925 (1986)

19. Ulmanen, I. et al., J. bacteriol., 162, 176-182 (1985)

20. Schein, C.H. et al., BIO/TECHNOLOGY, 4,719 (1986)

21. Rosenberg, M. et al., Ann. Rev. Genet., 13,319 (1979)

22. Moran Jr., C.P. et al., Mol. Gen. Genent., 186, 339-346 (1986)

23. Goldfarb D.S. et al., Nature 293,309 (1981)

24. Chang, S. et al., Mol. Gen. Genet., 168, 111-115 (1979)

25. Maniatis, T. et al., "Molecular Cloning", p 440, Cold Spring Harbor Laboratory (1982)

26. Tabak H.F. et al., Nucleic Acids Res., 5, 2321-2321 (1978)

27. Crea R. et al., Proc. Natl. Acad. Sci. USA, 75, 5765 (1978)

28. Goeddel, D.V. et al., Proc. Natl. Acad. Sci. USA, 76, 106

29. Ogasawara, N., Gene, 40, 145-150 (1985)

30. Chang J., FEBS Lett., 164, 307-313 (1983)

31. Walsmann P. et al., THREOBOSIS RESERCH 40, 563-569 (1985)

32. Hewick R.M. et al., J. Biol. Chem., 256, 7990-7999 (1981)

33. Chang et al., "Method is Enzymol.", 91, 41 (1983)

**Claims**

1.  A hirudin secretion plasmid in which a DNA sequence is ligated to a vector DNA, said DNA sequence being prepared by ligating a DNA fragment coding for a hirudin to a site immediately after the region coding for the promoter, ribosome binding region and secretion signal of the neutral protease gene of a Bacillus bacterium.

2.  A hirudin secretion plasmid according to Claim 1 wherein said Bacillus bacterium is Bacillus amyloliquefaciens.

3.  A hirudin secretion plasmid according to Claim 1 wherein said DNA sequence is the following DNA sequence:

5'GATCTTAACATTTTTCCCC

TATCATTTTTCCCGTCTTCAT

TTGTCATTTTTTCCAGAAAAA

ATCGTCATTCGACTCATGTCT

AATCCAACACGTCTCTCTCGG

CTTATCCCCTGACACCGCCCG

CCGACAGCCCGCATGGACGAA

TCTATCAATTCAGCCGCGGAG

TCTAGTTTTATATTGCAGAAT

GCGAGATTGCTGGTTTATTAT

AACAATATAAGTTTTCATTAT

TTTCAAAAGGGGGATTTATT

GTGGGTTTAGGTAAGAAATTG

TCTAGTGCTGTAGCCGCTTCC

TTTATGAGTTTAACCATCAGT

CTGCCGGGTGTTCAGGCCGTT

GTTTATACAGATTGCACAGAA

TCCGGACAAAATTTATGTTTA

TGTGAAGAATCTAATGTTTGT

GGACAAGGAAATAAATGTATT

TTAGGATCTGATGGAGAAAAA

AATCAATGTGTTACAGGAGAA

GGAACACCGAAACCGCAATCT

CATAATGATGGAGATTTTGAA

GAAATTCCTGAAGAATATTTA

CAA3'

4.  A hirudin secretion plasmid according to Claim 1 wherein said vector DNA is a plasmid which can be replicated in the genus Bacillus bacterium.

5.  A hirudin secretion plasmid according to Claim 1 wherein said plasmid which can be replicated with the aid of said Bacillus bacterium is pUB110.

6.  A transformed strain which has been transformed with one hirudin secretion plasmid selected from said hirudin secretion plasmids described in Claims 1 to 5.

7.  A transformed strain according to Claim 6 wherein a microorganism to be transformed is Bacillus subtilis.

8.  A method for preparing a hirudin which comprises the steps of cultivating said transformed strain described in Claim 7 in a culture medium, and then recovering said hirudin from the supernatant of said culture medium.

**Patentansprüche**

1.  Hirudin-Sekretionsplasmid, bei dem eine DNA-Sequenz in eine Vektor-DNA ligiert worden ist, wobei die DNA-Sequenz durch Ligation eines für Hirudin codierenden DNA-Fragments in eine Stelle direkt hinter der Region, die für den Promoter, die Ribosomen-Bindungsstelle und das Sekretionssignal des neutralen Proteasegens eines Bacillus-Bakteriums codiert, hergestellt worden ist.

2.  Hirudin-Sekretionsplasmid gemäß Anspruch 1, wobei das Bacillus-Bakterium Bacillus amyloliquefaciens ist.

3. Hirudin-Sekretionsplasmid gemäß Anspruch 1, wobei die DNA-Sequenz die folgende DNA-Sequenz ist:

```
5'GATCTTAACATTTTTCCCC

TATCATTTTTCCCGTCTTCAT

TTGTCATTTTTTCCAGAAAAA

ATCGTCATTCGACTCATGTCT

AATCCAACACGTCTCTCTCGG

CTTATCCCCTGACACCGCCCG

CCGACAGCCCGCATGGACGAA

TCTATCAATTCAGCCGCGGAG

TCTAGTTTTATATTGCAGAAT

GCGAGATTGCTGGTTTATTAT
```

AACAATATAAGTTTTCATTAT

TTTCAAAAGGGGGATTTATT

GTGGGTTTAGGTAAGAAATTG

TCTAGTGCTGTAGCCGCTTCC

TTTATGAGTTTAACCATCAGT

CTGCCGGGTGTTCAGGCCGTT

GTTTATACAGATTGCACAGAA

TCCGGACAAAATTTATGTTTA

TGTGAAGAATCTAATGTTTGT

GGACAAGGAAATAAATGTATT

TTAGGATCTGATGGAGAAAAA

AATCAATGTGTTACAGGAGAA

GGAACACCGAAACCGCAATCT

CATAATGATGGAGATTTTGAA

GAAATTCCTGAAGAATATTTA

CAA3'

4. Hirudin-Sekretionsplasmid gemäß Anspruch 1, wobei die Vektor-DNA ein Plasmid ist, das in der Gattung Bacillus-Bakterium repliziert werden kann.

5. Hirudin-Sekretionsplasmid gemäß Anspruch 1, wobei das Plasmid, das mit Hilfe des Bacillus-Bakteriums repliziert werden kann, pUB110 ist.

6. Transformierter Stamm, der mit einem Hirudin-Sekretionsplasmid transformiert worden ist, das aus den in den Ansprüchen 1 bis 5 beschriebenen Hirudin-Sekretionsplasmiden ausgewählt worden ist.

7. Transformierter Stamm gemäß Anspruch 6, wobei ein zu transformierender Mikroorganismus Bacillus subtilis ist.

8. Verfahren zur Herstellung von Hirudin, wobei das Verfahren die Schritte aufweist: Kultivieren des in Anspruch 7 beschriebenen Stammes in einem Kulturmedium und dann Gewinnen von Hirudin aus dem Überstand des Kulturmediums.

**Revendications**

1. Plasmide de sécrétion d'hirudine dans lequel une séquence d'ADN est ligaturée à un vecteur d'ADN, ladite séquence d'ADN étant préparée par ligature d'un fragment d'ADN codant pour une hirudine à un site situé immédiatement après la région codant pour le promoteur, la région de liaison au ribosome et le signal de sécrétion du gène d'une protéase neutre d'une bactérie Bacillus.

**2.** Plasmide de sécrétion d'hirudine selon la revendication 1, dans lequel ladite bactérie <u>Bacillus</u> est <u>Bacillus amyloliquefaciens.</u>

**3.** Plasmide de sécrétion d'hirudine selon la revendication 1, dans lequel ladite séquence d'ADN est la séquence d'ADN suivante:

5'GATCTTAACATTTTTCCCC

TATCATTTTTCCCGTCTTCAT

TTGTCATTTTTTCCAGAAAAA

ATCGTCATTCGACTCATGTCT

AATCCAACACGTCTCTCTCGG

CTTATCCCCTGACACCGCCCG

CCGACAGCCCGCATGGACGAA

TCTATCAATTCAGCCGCGGAG

TCTAGTTTTATATTGCAGAAT

GCGAGATTGCTGGTTTATTAT

AACAATATAAGTTTTCATTAT

TTTCAAAAAGGGGGATTTATT

GTGGGTTTAGGTAAGAAATTG

TCTAGTGCTGTAGCCGCTTCC

TTTATGAGTTTAACCATCAGT

CTGCCGGGTGTTCAGGCCGTT

GTTTATACAGATTGCACAGAA

TCCGGACAAAATTTATGTTTA

TGTGAAGAATCTAATGTTTGT

GGACAAGGAAATAAATGTATT

TTAGGATCTGATGGAGAAAAA

AATCAATGTGTTACAGGAGAA

GGAACACCGAAACCGCAATCT

CATAATGATGGAGATTTTGAA

GAAATTCCTGAAGAATATTTA

CAA3'

4. Plasmide de sécrétion d'hirudine selon la revendication 1, dans lequel ledit vecteur d'ADN est un plasmide qui peut se répliquer dans une bactérie du genre Bacillus.

5. Plasmide de sécrétion d'hirudine selon la revendication 1, dans lequel ledit plasmide qui peut se répliquer à l'aide de ladite bactérie Bacillus est pUB110.

6. Souche transformée qui a été transformée avec un plasmide de sécrétion d'hirudine choisi parmi lesdits plasmides de sécrétion d'hirudine décrits dans les revendications 1 à 5.

7. Souche transformée selon la revendication 6, dans laquelle le microorganisme à transformer est Bacillus subtilis.

8. Méthode de préparation d'une hirudine qui comprend les étapes de culture de ladite souche transformée décrite dans la revendication 7 dans un milieu de culture, puis de récupération de ladite hirudine à partir du surnageant dudit milieu de culture.

# FIG.1

Synthesized Oligoncleotide

HpaⅡ      StuI    BamHI

GGGTGTTCAGGCCTG
CCACAAGTCCGGACCTAG

— BamHI
— HpaⅡ

— T₄ Ligase

| HpaⅡ | | StuI | | BamHI | | |
|---|---|---|---|---|---|---|
| GTG GGT---CCG GGT GTT CAG GCC | | | | TGG ATC CCC | | CTT------ |
| secretion signal coding region | | | | junction region | | α-amylase -gene |

pNPA225
7.8kb

SD
promoter
Δprotease

# F I G.2

synthesized DNA

```
  1                      ΔH                              10
     Val Val Tyr Thr Asp Cys Thr Glu Ser Gly
5' CC GTT GTT TAT ACA GAT TGC ACA GAA TCC GGA TG 3'
3' GG CAA CAA ATA TGT CTA ATG TGT CTT AGG CCT ACC TAG 5'
                                         AccⅢ
```

# F I G.3

```
         1                                      10
        Val  Val  Tyr  Thr  Asp  Cys  Thr  Glu  Ser  Gly
5' CC   GTT  GTT  TAT  ACA  GAT  TGC  ACA  GAA  TCC  GGA  TG 3'
3' GG   CAA  CAA  ATA  TGT  CTA  ATG  TGT  CTT  AGG  ACC  TAG 5'
                                              AccIII cleavage site
```

# F I G.4

EcoRI
BamHI
H
AccIII
p4014
4.3kb

EcoRI
BamHI
HindIII
pUC13
3.1kb

EcoRI, BamHI          EcoRI, BamHI

Ligation

EcoRI    BamHI
HindIII
H
p3009